# EUROPEAN PATENT APPLICATION

(11) **EP 4 356 926 A1**
(43) Date of publication of application: **24.04.2024**
(21) Application number: 22735764.7
(22) Date of filing: 06.06.2022
(51) Int. Cl.: A61K 39/395, C07K 16/28

(54) **USE OF MONOCLONAL ANTIBODIES AGAINST THE EPIDERMAL GROWTH FACTOR RECEPTOR IN THE TREATMENT OF PATIENTS WITH ACUTE HYPOXAEMIC RESPIRATORY FAILURE**

(30) Priority: 14.06.2021 CU 20210046
(71) Applicant: Centro de Inmunologia Molecular, La Habana, Habana 11300 (CU)
(72) Inventor: CROMBET RAMOS, Tania, Boyeros La Habana Habana 10800 (CU); RAMOS SUZARTE, Mayra, La Habana Habana 11300 (CU); SAAVEDRA HERNÁNDEZ, Danay, La Habana Habana 10800 (CU); AÑÉ KOURÍ, Ana Laura, La Habana Habana 10400 (CU); PÉREZ RODRÍGUEZ , Rolando, La Habana Habana 10700 (CU); DÍAZ LONDRES, Henrry, La Habana Habana 10900 (CU); JIMÉNEZ ARMADA, Jorge, La Habana Habana 10700 (CU); LEÓN MONZÓN, Kalet, La Habana Habana 17100 (CU); ABDO CUZA, Anselmo Antonio, La Habana Habana 10300 (CU)
(74) Representative: Capitán García, Maria Nuria
(86) International application number: PCT/CU2022/050006
(87) International publication number: WO 2022/262879

(57) **Abstract**

The present invention relates to the fields of biotechnology and medicine and provides the use of monoclonal antibodies against the epidermal growth factor receptor in the treatment of diseases of infectious origin that leading to a hypoxemic acute respiratory failure.

## Description

### FIELD OF THE INVENTION

The present invention relates to the fields of biotechnology and medicine and particularly to the use of monoclonal antibodies (MAbs) against the epidermal growth factor receptor in the treatment of patients with hypoxemic acute respiratory failure.

### BACKGROUND

Hypoxemic acute respiratory failure is the leading cause of emergency admissions. The mortality rate is almost 20%. The most frequent etiologies are pneumonia, neurological causes, non-pulmonary sepsis and chronic obstructive pulmonary disease. The disease is characterized by dyspnea at rest, arterial O2 pressure (PO2) less than 60 mmHg, and/or arterial CO2 pressure (PCO2) greater than 45 mmHg (Joao D et al. (2019) J Crit Care 49: 84-91.; Piriano T et al. (2019) Respir Care. 64(6):638-646).

Respiratory failure can progress to acute respiratory distress syndrome (ARDS), which is a life-threatening condition caused by lung disease or not. (Butt Y and cols. (2016) Arch Pathol Lab Med.;140(4):345-50). It is the result of a systemic inflammatory disease that results in pulmonary infiltrate, edema, and hypoxia. This process is characterized at the cellular level by loss of the integrity of the alveolar-capillary membrane, excessive infiltration of neutrophils, and release of pro-inflammatory cytokines and chemokines. (Shan X and cols. (2017) Oncotarget;8(16):26648-61). At the pathological level, there is intra-alveolar and interstitial edema in the acute phase, proliferation of type II alveolar cells in the proliferative phase, and fibrosis in the more advanced stages. (Poletti V and cols. (2010) Pathologica. 102(6):453-63)

Clinically, it is manifested by hypoxemia, with bilateral opacities on chest X-ray, associated with decreased compliance. Morphologically, diffuse alveolar damage is seen in the acute phase of ARDS. Mortality from ARDS remains high, ranging between 35% and 46%.(Ferguson ND y cols. (2012) Intensive Care Med.;38(10):1573-82; Huppert LA y cols. (2019) Semin Respir Crit Care Med.;40(1):31-9). Survivors may have persistent morbidity that is associated with significant impairment in quality of life (Butt Y y cols. (2016) Arch Pathol Lab Med.;140(4):345-50; Huppert LA y cols. (2019) Semin Respir Crit Care Med.;40(1):31-9; Fan E y cols. (2018) JAMA.;319(7):698-710).

Although some ventilatory strategies have improved the outcomes of acute lung injury, most therapies with corticosteroids or surfactants have shown controversial results. (Fan E and cols. (2018) JAMA.;319(7):698-710).

Epidermal growth factor receptor (EGFR) is a 170 kDa membrane glycoprotein, which controls fundamental cellular processes, including cell migration, cell cycle, cell metabolism and survival, as well as cell proliferation and differentiation. (Shan X and cols. (2017) Oncotarget;8(16):26648-61; Mendelsohn J, Baselga J. (2006) Semin Oncol.;33(4):369-85; Saba NF and cols. (2006) Oncology;20(2):153-61). The physiological role of EGFR is to regulate epithelial tissue development and homeostasis. (Mendelsohn J, Baselga J. (2006) Semin Oncol.;33(4):369-85).

EGFR and its ligands are upregulated in multiple tumors of epithelial origin, including non-small cell lung cancer, gliomas, tumors of the head and neck, cervix, and esophagus (Mendelsohn J, Baselga J. (2006) Semin Oncol.;33(4):369-85). EGFR overexpression in tumors correlates with increased metastasis, decreased survival, and poor prognosis. Its intracellular domain is associated with specific protein tyrosine kinase activity and its over-expression by cells alters cell cycle regulation, blocks apoptosis, promotes angiogenesis, increases motility, cell adhesiveness and metastization. (Mendelsohn J, Baselga J. (2006) Semin Oncol.;33(4):369-85, Saba NF and cols. (2006) Oncology;20(2):153-61). Several drugs have been developed with the purpose of blocking signaling through the EGFR, such as tyrosine kinase inhibitors (TKIs) including gefitinib, erlotinib, and osimertinib, and anti-EGFR MAbs such as cetuximab, panitumumab, and nimotuzumab. (Mendelsohn J, Baselga J. (2006) Semin Oncol.;33(4):369-85, Saba NF and cols. (2006) Oncology;20(2):153-61; Perez R and cols. (2011) Clinical Effects. Cancers (Basel);3(2):2014-31; Smith D and cols. (2009) Bull Cancer.;96 Suppl:S31-40).

Acute respiratory insufficiency results in a positive regulation and over-expression of EGFR. In essence, EGFR activation results in more severe lung pathology. The main node in pulmonary fibrosis is the EGFR pathway, which controls many cascades of cell proliferation, mucus secretion, inflammatory response, and tissue repair. (Venkataraman T, Frieman MB. (2017) Antiviral Res.;143:142-50).

Despite this evidence, in mice there are conflicting reports regarding the effect of EGFR TKIs on fibrosis. Suzuki and his colleagues reported increased fibrosis after gefitinib treatment. (Suzuki H and cols. (2003) Cancer Res.;63(16):5054-9), while Ishii and colleagues observed a decrease in fibrosis (Ishii Y, Fujimoto S, Fukuda T. (2006) Am J Respir Crit Care Med.;174(5):550-6). The causes of this discrepancy are unclear, but variations in mouse strain and duration of gefitinib treatment could explain the differences. (Suzuki H and cols. (2003) Cancer Res.;63(16):5054-9; Ishii Y, Fujimoto S, Fukuda T. (2006) Am J Respir Crit Care Med.;174(5):550-6).

In patients with lung cancer, TKIs can induce acute hypoxemic respiratory failure secondary to interstitial pneumonitis. This adverse event occurs with a low frequency of 1.1 to 2.2%, but can be fatal, accounting for 58% of all EGFR-TKI treatment-related deaths. (Ohmori T and cols. (2021) Int J Mol Sci.;22(2):792). Patients in Japan show significantly higher incidence of pneumonitis, of unknown cause at this time (Suh CH and cols. (2018) Lung Cancer;123:60-9).

Surprisingly, the inventors of the present invention, when administering nimotuzumab to patients suffering from hypoxemic acute respiratory failure or ARDS, observed improvements in the ventilatory parameters, in inflammation, in the lung lesions, and increased survival.

### BRIEF DESCRIPTION OF THE INVENTION

In one embodiment, the present invention relates to the use of MAbs directed against EGFR in the treatment of diseases that lead to hypoxemic acute respiratory failure of infectious origin and in particular those classified as ARDS.

In a particular embodiment of the invention, acute respiratory failure is of infectious origin and may be caused by microorganisms selected from the group comprising: coronavirus, rinovirus, citomegalovirus, Epstein Barr, influenza virus, respiratory syncytial virus, *Streptococcus pyogenes, Streptococcus pneumoniae, Haemophilus influenza, Escherichia coli, Pseudomonas aeruginosa* , *Proteus mirabilis* , *Enterobacter spp , Klebsiella spp* , *Acinetobacter spp* , *Staphylococcus spp* , *Serratia sp Cryptococcus neoformans* , *Rhizopus sp* , *Aspergillus spp* , *Pneumocystis spp* , *Mucor sp* Y *Rhizomucor spp.*

Among the anti-EGFR MAbs contemplated by the present invention that can be used in the treatment of hypoxemic acute respiratory failure or ARDS are nimotuzumab, cetuximab and panitumumab.

In another embodiment, the present invention is related to a method of treating a subject that comprises the administration of an anti-EGFR mAb intravenously or subcutaneously in a dose range of 1 mg/Kg of weight to 6 mg /Kg of weight for at least two times and up to a maximum of five times spaced between 72 and 168 hours.

### DETAILED DESCRIPTION OF THE INVENTION

### Pharmaceutical compositions

The mAb against EGFR that are used in the present invention are administered as part of pharmaceutical compositions containing the mAb as active ingredient and pharmacologically appropriate excipients for parenteral use. In particular, among these MAbs are nimotuzumab whose sequence is described in US patent 5,891,996, cetuximab described in US patent 6,217,866 B1 and panitumumab whose sequence is described in US 5,558,864 B1

### Therapeutic application and treatment methods

The present invention describes the use of anti-EGFR antibodies in the treatment of acute hypoxemic respiratory failure or ARDS. In particular, the use is described for the case of patients where acute respiratory failure or respiratory distress syndrome is caused by an infectious agent, for example, a virus, a bacterium or a fungus. In particular, anti-EGFRs can be used to treat acute respiratory failure or ARDS induced by viruses such as coronaviruses, rhinovirus, cytomegalovirus, Epstein Barr virus, influenza virus, and respiratory syncytial virus. In the case of bacterial infections, those caused by streptococci, pneumococci or staphylococci, *Haemophilus influenzae, Escherichia coli, Pseudomonas aeruginosa, Proteus mirabilis, Enterobacter spp, Klebsiella spp, Acinetobacter spp* and *Serratia spp.* When the infectious agent is a fungus, this may be the *Cryptococcus neoformans, Rhizopus spp, Aspergillus spp, Pneumocystis spp, Mucor spp* either *Rhizomucor spp.*

The use of anti-EGFR antibodies as described in the present invention improves the symptoms and signs of acute hypoxemic respiratory failure or acute respiratory distress syndrome.

The reduction of these symptoms allows an improvement and better management of the patient by the medical team. A particular advantage of the proposed treatment is the non-induction of immunodeficiency in the patient, unlike other conventional therapies based on steroids or other immunosuppressants. The preservation of immuno-competence in the treated patient reduces the possibility of emergence of other opportunistic infections, very common in severe or critical patients.

Patients receiving anti-EGFR antibody therapy are preferably admitted to an intensive or intermediate care ward, although they may also be in a conventional ward. They show at least the following symptoms: dyspnea at rest, arterial O2 pressure (PO2) less than 60 mmHg and/or arterial CO2 pressure (PCO2) greater than 45 mmHg, lower oxygen saturation (SO2) < 94% on room air at sea level or need for oxygen therapy to maintain SO2 > 93%, ratio of arterial pressure of oxygen/inspired fraction of oxygen (PaO2/FiO2) < 300 mm Hg, respiratory rate > 30 inspirations/min, or presence of infiltrate in more than 50% of both lung fields. The confirmatory diagnosis is established considering the parameters of the arterial blood gases and the radiological findings, either X-rays, pulmonary ultrasound or Computed Axial Tomography.

Other signs and symptoms include dyspnea, high respiratory rate, tachycardia, cyanosis, confusion, fatigue, hypotension, dry cough, fever, 38°C, headache, neurological manifestations. A variation is observed in different laboratory parameters such as: increase above normal limits in the values of D-dimer, ferritin, LDH, C-reactive protein, IL-6.

The anti-EGFR MAbs will be applied to patients intravenously or subcutaneously, in a dose range between 1 mg/Kg of weight and 6 mg/Kg of weight, which correspond approximately to between 50 and 600 mg total, respectively. Preferably, in the case of the anti-EGFR antibody nimotuzumab, doses between 1.42 mg/kg and 2.85 mg/kg will be used. These MAbs will be administered at least twice to the patient and with a maximum of 5 doses. The time between two consecutive administrations will be between 72 and 168 hours. In the case of nimotuzumab, the preferred schedule is two to three doses spaced every 72 hours, using a loading dose of 200 mg and subsequent doses of 100 mg. The dose and schedule to be used for the different anti-EGFR MAbs may be adjusted by evaluating their ability to recognize EGFR.

Another object of the present invention is the serial or concomitant combination of anti-EGFR antibodies, with antiviral therapies or antibiotics. Among the possible antibiotics to be combined are, among others, those of the cephalosporin family such as ceftriaxone, macrolides such as azithromycin, glycopeptides such as vancomycin, beta-lactams such as meropenem and oxazolidinones such as linezolid. Among the possible antivirals to be combined will be, among others, type 1 and type 2 interferons, antiretrovirals used in the treatment of HIV such as remdesivir. The antibiotic or antiviral regimens to be used will be those conventionally used for each drug, while the regimen for the use of the anti-EGFR antibody will respond to what has been described above. In addition, the object of the present invention is the serial or concomitant combination of anti-EGFR antibodies, with other anti-inflammatory therapies or anticoagulant therapies such as steroids or other immunomodulatory drugs and with anticoagulant therapies such as low molecular weight fractionated heparin or heparin, heparin sodium, unfractionated.

### BRIEF DESCRIPTION OF THE FIGURES

**Figure 1****.** Axial chest computed tomography scans of 3 patients treated with nimotuzumab. Sequential images A) on admission (column, B) at discharge and C) follow-up (30-60 days after discharge).
**Figure 2****.** Behavior over time of the PaO2/FiO2 ratio, before and after the administration of nimotuzumab.
**Figure 3****.** Variation in lactate dehydrogenase (LDH) enzyme concentrations before and after nimotuzumab administration.
**Figure 4****.** Radiological evolution of three patients before and after treatment with nimotuzumab: A) Patient JMTR, B) Patient JHV, C) Patient MESS.
**Figure 5****.** Global evolution of patients with COVID-19 treated with nimotuzumab.
**Figure 6****.** D-dimer values in patients treated with nimotuzumab.

### EXAMPLES

### Example 1. The use of nimotuzumab reduces the SARS-CoV-2 mortality rate in patients with hypoxemic acute respiratory failure.

Fourteen patients diagnosed with SARS-CoV-2 with clinical, radiological, or laboratory evidence of hypoxemic acute respiratory failure or respiratory distress syndrome received a 200-mg dose of the monoclonal antibody nimotuzumab intravenously. All patients received a second dose equivalent to 100 mg, 72 hours after the first.

To evaluate the impact on the use of the investigational product, a group of patients positive for SARS-CoV-2, classified as severe and who received concurrent treatment with steroids and anticoagulants in 20 hospitals in Cuba, was selected as a control.

**Table 1. Mortality of seriously ill patients treated with nimotuzumab.**

| | | Deceased | | Total |
|---|---|---|---|---|
| | | No | Yes | |
| nimotuzumab | Count | 13 | 1 | 14 |
| | % in each group | 92.85 | 7.14 | 100 % |
| Control | Count | 49 | 15 | 64 |
| | % in each group | 76,6% | 23,4% | 100,0% |
| Total | Count | 64 | 16 | 80 |
| | % in each group | 80 % | 20 % | 100 % |

Table 1 shows that the use of nimotuzumab reduced the mortality rate by 16% compared to the control group. Internationally, the survival rate for severe COVID-19 patients ranges from 77% to 81% after the use of best supportive therapy including anticoagulation and steroids and an anti-IL6 receptor antibody in some patients ( tocilizumab)(Group RC (2021) Lancet;397(10285): 1637-45; Rosas IO y cols. (2021) N Engl J Med.;384(16):1503-16). The recovery data of nimotuzumab (92.85%) in combination with the therapy established in the Cuban protocol compare very favorably with Cuban control patients (77%) and multinational trials (77-81%).

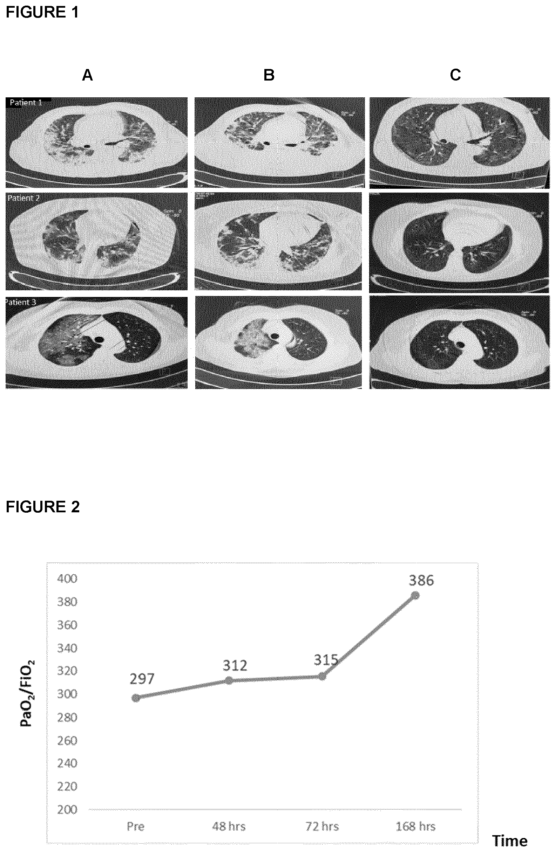

Axial chest computed tomography scans were performed to three patients treated with nimotuzumab. Figure 1A and B show extensive areas of ground glass opacities, airspace consolidation in exudative phase and decreased lung volumes in organizing and fibrotic phases. In contrast, Figure 1C shows that the 3 patients showed resolution of the lung inflammatory lesions and no sign of fibrosis.

### Example 2. The use of nimotuzumab improves the ventilatory parameters of treated patients.

In 10 seriously ill COVID-19 patients, the ratio between arterial oxygen pressure (PaO2) and inspired fraction of oxygen (FiO2) was calculated as one of the ventilation parameters. Figure 2 shows the behavior of this ratio over time, before nimotuzumab and after 2 doses of the antibody separated by 72 hours. As seen in the Figure, patients showed improvement in pulmonary ventilation over time.

### Example 3: Treatment with nimotuzumab improves the inflammatory parameters of treated patients.

As a measure of inflammation in 8 severe COVID-19 patients, LDH and IL-6 were measured before and after treatment with nimotuzumab, the latter by ELISA (Quantikine). All patients had steroid treatment before administration of the monoclonal antibody.

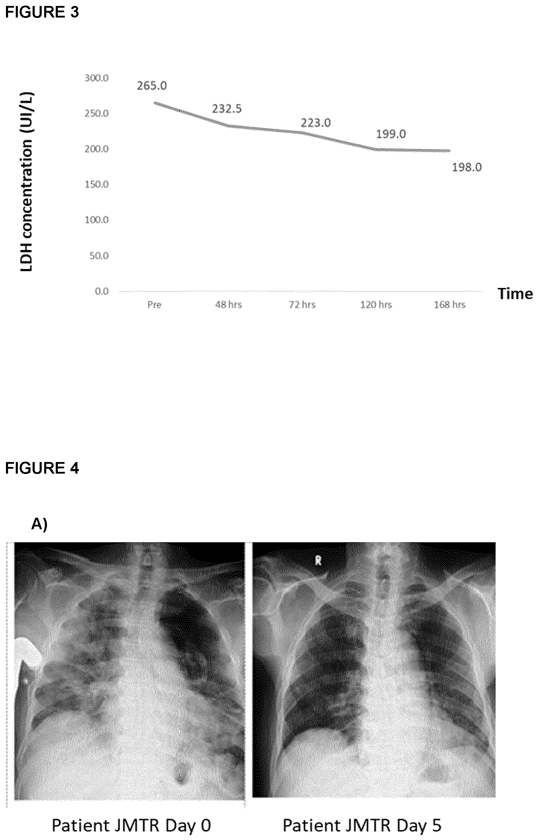

Figure 3 shows that a decrease in LDH occurs after 2 doses of MAb nimotuzumab. Table 2 shows the concentrations of IL-6 before and at different times of treatment with nimotuzumab.

**Table 2. Variation of IL-6 values before and at different times of treatment with nimotuzumab.**

| Patients | Day 0 | 48h | 72h | D5 | D7 |
|---|---|---|---|---|---|
| JAS | 50.75 | 10.20 | 3.29 | 4.11 | 0.29 |
| MAVO | 21.29 | 24.93 | 9.75 | 0.00 | 0.00 |
| HPH | 23.47 | 11.29 | 58.93 | 17.29 | 18.38 |
| MESS | 112.93 | 77.38 | 45.11 | 26.75 | 16.56 |
| JMTR | 67.29 | 0.00 | 0.00 | 35.93 | 0.00 |
| MVH | 8.93 | 34.93 | 6.29 | 0.00 | 10.75 |
| RGR | 40.56 | 47.56 | | 43.65 | 47.02 |
| MAST | 15.75 | 190.38 | 130.75 | 503.84 | |

In 5 of the 8 patients, the decrease in serum IL-6 concentration on day 0 and day 7 was significant (p=0.048 Friedman's test, D0vsD7 p=0.03 Dunn's test). In patient MVH, IL-6 increases at 48h (although it remains below average) and then decreases, while in the RGR patient, IL-6 concentrations remain stable. In these 7 patients, the evolution is classified as favorable, given the marked increase trend as part of the natural history of the disease. In the MAST patient, IL-6 increased which correlated with the torpid course of the disease.

### Example 4. Nimotuzumab treatment improves the area affected by multifocal interstitial pneumonia.

In 8 patients with different times of evolution of COVID-19 after 2 doses of nimotuzumab, the percentage of affected area of both lung fields (right lung and left lung) was calculated. These results are shown in Table 3.

**Table 3. Percentage of lung volume affected.**

| Pacients | Lung area affected before nimotuzumab | Lung area affected one week after nimotuzumab |
|---|---|---|
| MESS | 50 % | 25 % |
| JMTR | 47.5 % | 4% |
| MNVH | 35 % | 15% |
| JHV | 50 % | 35 % |
| EVT | 47.5 % | 15% |
| CTN | 30 % | 40 % |
| MAST | 30 % | 40 % |
| GGA | 27.5 % | 27.5 % |

| | | |
|---|---|---|
| * The results are shown as the sum of both lung fields. | | |

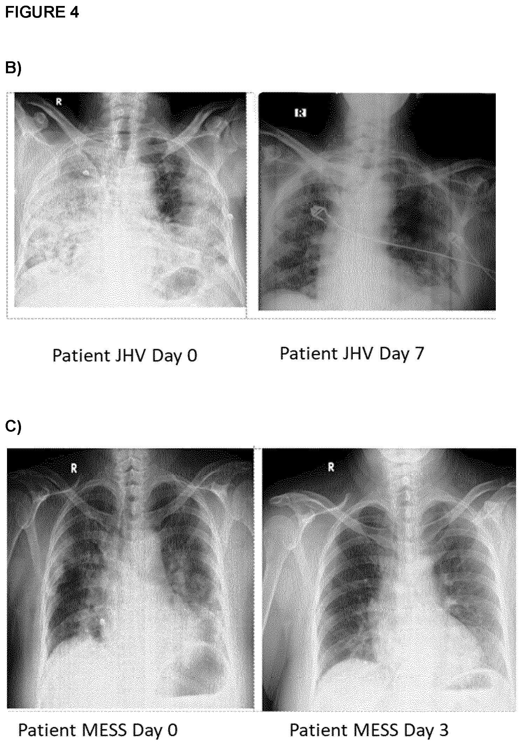

Overall, the use of nimotuzumab improved the affected area in 62.5% of treated patients, 168 hours after the first dose of monoclonal antibody. In 1 patient, the affected area of both lung fields did not change, while in 2 patients the affected area only increased by 10% on the seventh day of evolution. Figure 4 shows the radiological evolution of three of these patients before and after treatment with nimotuzumab.

### Example 4. Patients with ARDS caused by COVID-19 have clinical improvement after treatment with nimotuzumab.

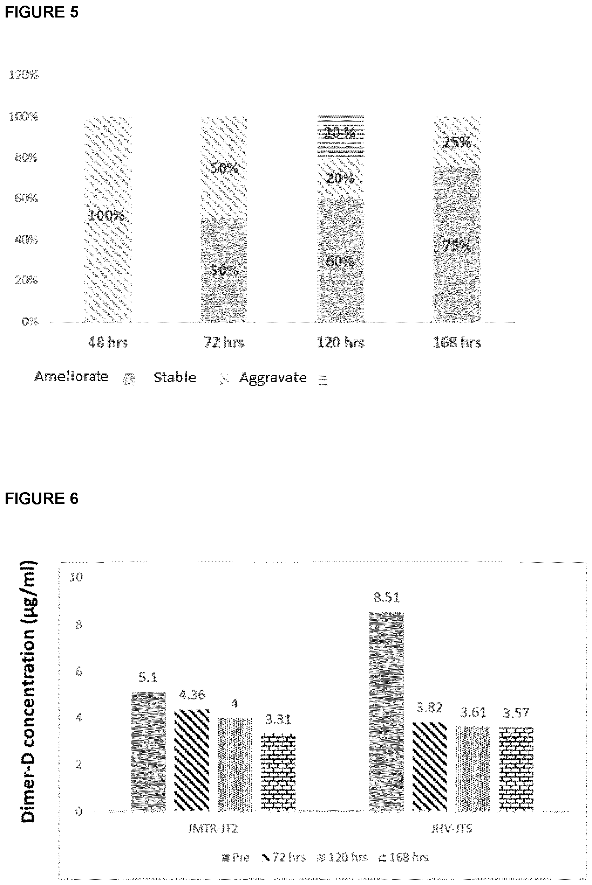

An evaluation of their global evolution (clinical, radiological and laboratory), considering the improvement in some of the parameters that are routinely measured in each of these evaluations. Figure 5 shows that overall, 75% of patients showed signs of clinical improvement one week after the first dose of nimotuzumab.

### Example 5. Nimotuzumab treatment produces a decrease in D-dimer in severe COVID-19 patients.

Two patients with COVID-19, treated with nimotuzumab and the rest of the drugs included in the Cuban protocol for managing this disease, had their D-dimer measured. Figure 6 shows that both patients had a significant reduction in D-dimer, after the combination of nimotuzumab with the rest of the therapies provided for COVID-19, in the Cuban protocol. It is necessary to clarify that both patients reached high levels of D-dimer, even under treatment with anticoagulant (fractionated heparin), steroids and other immunomodulators.

## Claims

1. Use of a monoclonal antibody (MAb) against the epidermal growth factor receptor in the treatment of diseases leading to hypoxemic acute respiratory failure.

2. Use according to claim 1 wherein the hypoxemic acute respiratory failure is an acute respiratory distress syndrome.

3. Use according to any of claims 1-2 wherein the diseases have infectious origin.

4. Use according to claim 3 wherein the infectious diseases are caused by the microorganism selected from the group comprising:
- coronavirus, rinovirus, citomegalovirus, Epstein Barr, influenza virus, respiratory syncytial virus, *Streptococcus pyogenes, Streptococcus pneumoniae, Haemophilus influenzae, Escherichia coli, Pseudomona aeruginosa,* Proteus mirabilis, Enterobacter spp, Acinetobacter spp, Staphilococcus spp, Klebsiella Serratia spp, Cryptococcus neoformans, Rhizopus spp, Aspergillus spp, Pneumocystis spp, Mucor spp y Rhizomucor spp.

5. Use according to any of claims 1-4 wherein the MAb against the EGFR are selected from the group comprising:
- nimotuzumab, cetuximab and panitumumab.

6. A method of treatment of the subject in need thereof comprising the administration of an anti-EGFR MAb by intravenous or subcutaneous route in a range from 1 to 6 mg/Kg of body weight.

7. The method of claim 6 wherein the anti-EGFR MAb is administered to the subject at least twice up to a maximum of five with time elapsed between two consecutive administrations between 72 and 168 hours.
